Europäisches Patentamt

(19) European Patent Office

Office européen des brevets

(11) Publication number : **0 466 650 A2**

(12) # EUROPEAN PATENT APPLICATION

(21) Application number : **91810526.3**

(22) Date of filing : **05.07.91**

(51) Int. Cl.⁵ : **A61K 33/00, A61K 45/06**

(30) Priority : **11.07.90 US 551717**

(43) Date of publication of application :
**15.01.92 Bulletin 92/03**

(84) Designated Contracting States :
**BE DE DK ES FR GB GR IT**

(71) Applicant : **WARNER-LAMBERT COMPANY**
**201 Tabor Road**
**Morris Plains New Jersey 07950 (US)**

(72) Inventor : **Granger, Colin D.**
**615 Hillside Road**
**Chester, New Jersey 07930 (US)**

(74) Representative : **Silbiger, Jakob, Dr.**
**c/o CAPSUGEL AG Fabrikmattenweg 2-4**
**CH-4144 Arlesheim (CH)**

(54) **Improvements in non-steroidal anti-inflammatory therapy.**

(57)    Inflammation is treated by administration in a multiple dose regimen of a non-steroidal systemic anti-inflammatory agent concomitantly with a nontoxic metal base or basic salt. The metal base or basic salt can be a hydroxide, sulfate, carbonate, bicarbonate, subcarbonate, or trisilicate. The metal can be aluminum, sodium, magnesium, potassium, or bismuth. Aluminum hydroxide is preferred.

EP 0 466 650 A2

Various non-steroidal anti-inflammatory agents operate systemically through inhibition of the biosynthesis of prostaglandins, specifically $PGE_2$, The mechanism of this action is believed to involve inhibition of the enzyme cyclooxygenase.

Non-steroidal anti-inflammatory agents of this type fall into various classes based broadly on structure. Csáky and Barnes in "Cutting's Handbook of Pharmacology," Appleton-Century-Crofts, Norwalk, CN (1984) describe such non-steroidal anti-inflammatory agents as including, among others, fenamic acid derivatives, indene derivatives, and ibufenac derivatives. While by no means free of overlap and while other classifications exist (see, for example, Sunshine et al., U.S. Patent No. 4,552,899), that of Csáky and Barnes is useful for present purposes.

Fenamic acid derivatives are broadly classified as o-anilino derivatives of benzoic, phenylacetic, and nicotinic acids and are defined by Csáky and Barnes as including flufenemic acid, mefenamic acid, meclofenamic acid, clonixeril, clonixin, flunixin, and diclofenac, including the pharmaceutically acceptable salts thereof.

Indene derivatives generally are acetic acid, propionic acid, and benzoic acid derivatives containing a pyrrole (or a pyrazole, pyrroline or pyrrolidine) ring and are defined by Csáky and Barnes as including indomethacin, carprofen, etodolac, fendosal, indoprofen, prodolic acid, sermetacin, zidometacin, and zomeprirac, including the pharmaceutically acceptable salts thereof. Compounds not so classified by Csáky and Barnes but related in being acetic acid, propionic acid, or benzoic acid, derivatives and here so classified include tolmetin, sulinac, cicloprofen, and cinchophen, including the pharmaceutically acceptable salts thereof.

Ibufenac derivatives are acetic and propionic acid derivatives bearing a substituted aromatic hydrocarbon ring, such as phenyl or naphthyl, and are defined by Csáky and Barnes as including iflunisal, fenoprofen, ibuprofen, naproxen, alclofenac, amfenac, cliprofen, fenclofenac, fenclorac, fluprofen, ketoprofen, naproxol, fenbufen, and ibufenac, including the pharmaceutically acceptable salts thereof.

The prostaglandin, $PGE_2$, whose biosynthesis is thus inhibited also is involved in the normal regenerative process for the gastric mucosa. Thus while the inhibitory effect on $PGE_2$ biosynthesis is desirable at the site of inflammation, it also is responsible for the gastric irritation associated with non-steroidal anti-inflammatory therapy. Damage to the stomach as a result this inhibition of prostaglandin biosynthesis generally is minimal with individual doses, although some discomfort may arise, but administration of a non-steroidal anti-inflammatory agent in a multiple dose regimen during long term therapy, as in the treatment of chronic inflammation, can overwhelm the body's ability to adjust. Thus Graham (Gastroenterology,, 96, No. 2, 675 (1989)) noted that 10% of patients on long term therapy with non-steroidal $PGE_2$ inhibiting anti-inflammatory agents have a point prevalence of ulcer disease 5 to 10 times higher than those not taking non-steroidal anti-inflammatory agents.

Charlet et al., J. Clin. Pharmacol. (1985); 25: 564, describe a number of models for studying cytoprotection and noted that natural and synthetic prostaglandins reduced the incidence of gastric ulcers and erosion of the gastric mucosa caused by indomethacin, no doubt by compensating for the inhibition of prostaglandin biosynthesis through introduction of exogenous prostaglandins.

According to the present invention, there is provided the use of a nontoxic prostaglandin-stimulating metal base or basic salt in the manufacture of a medicament for the treatment of inflammation.

The present invention further provides said use, wherein the medicament is administered in close temporal proximity to a multiple dose regimen of a non-steroidal systemic anti-inflammatory agent which inhibits biosynthesis of prostaglandins and which is selected from the group consisting of fenamic acid derivatives, indene derivatives, and ibufenac derivatives.

The present invention still further provides a kit of parts useful in the treatment of inflammation, comprising:
a) a medicament, for the treatment of inflammation, as obtained from the use of a nontoxic prostaglandin-stimulating metal base or basic salt;
b) a multiple dose regimen of a non-steroidal systemic anti-inflammatory agent selected from the group consisting of fenamic acid derivatives, indene derivatives, and ibufenac derivatives; and
c) instructions for the close temporal administration of (a) and (b).

The metal can be aluminum, magnesium, sodium, potassium, or bismuth. The metal base or basic salt can be the hydroxide, sulfate, carbonate, bicarbonate, subcarbonate, or trisilicate.

The non-steroidal anti-inflammatory agent is administered in the same dosages as are conventionally used when administered by itself.

The non-steroidal anti-inflammatory agents in most cases are carboxylic acids and can be administered in the form of a pharmaceutically acceptable salt. Pharmaceutically acceptable salts include bases such as the alkali metals, ammonium, and mono-, di- and trisubstituted amines, such as for example the sodium, potassium, lithium, ammonium, trimethylammonium, triethanolammonium, pyridinium, and substituted pyridinium salts. The sodium salt is preferred.

The metal base or basic salt utilized in the present invention is administered in an amount ranging from about 25% to about 100% by weight of the amount of the non-steroidal antiinflammatory agent. The two components can be formulated in a unitary pharmaceutical formulation or administered separately but in temporal proximity, that is, sufficiently close to the administration of the anti-inflammatory agent to be present in the stomach at the same time. The metal base or basic salt can be administered prior to the administration of the non-steroidal anti-inflammatory agent but it is important further administration be made at the time the non-steroidal anti-inflammatory agent is administered.

Conventional pharmaceutical formulations which permit release of the active ingredient in the stomach such as tablets, capsules, suspensions, and the like are employed. It is important that the metal base or basic salt is released in the stomach in order that it may perform its cytoprotective action.

In the pharmaceutical compositions and methods of the present invention, the foregoing active ingredients will typically be administered in admixture with suitable pharmaceutical diluents, excipients or carriers (collectively referred to herein as "carrier" materials) suitably selected with respect to the intended form of administration, i.e., oral tablets, capsules, elixirs, syrups, suspensions, etc. and consistent with conventional pharmaceutical practices. For instance, for oral administration in the form of tablets or capsules, the active drug components may be combined with any oral nontoxic pharmaceutically acceptable inert carrier such as lactose, starch, sucrose, cellulose, magnesium stearate, dicalcium phosphate, calcium sulfate, mannitol, ethyl alcohol (liquid forms) and the like. Moreover, when desired or necessary, suitable binders, lubricants, disintegrating agents and coloring agents can also be incorporated into the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, metallic stearates, stearic acid, PEG 4000, waxes, sodium acetate, sodium chloride, etc. Disintegrators include without limitation, starch, methylcellulose, agar, bentonite, guar gum, corn starch, cross-linked sodium CMC, cross-linked PVP, swellable cellullosics, etc. Sweetening and flavoring agents and preservatives can also be included where appropriate.

Of course, in addition, the compositions of the present invention may be formulated in sustained release form.

The following examples are for purposes of illustrating the present invention and are not intended in any way to limit the scope thereof which is defined by the appended claims.

EXAMPLE 1

Three possible tablet formulations utilizing the features of the present invention are as follows.

| Ingredient | Mg./tablet | | |
|---|---|---|---|
| | 1 | 2 | 3 |
| Indomethacin | 25.0 | -- | -- |
| Naproxen | -- | 250.0 | -- |
| Ibuprofen | -- | -- | 300.0 |
| Aluminum OH | 200.0 | -- | -- |
| Magnesium Trisilicate | -- | 500.0 | -- |
| Bismuth Subcarbonate | -- | -- | 500.0 |
| Lubricant | 2.0 | 4.0 | 4.0 |
| Silicon Dioxide | 1.6 | 3.25 | 3.25 |
| Microcrystalline Cellulose | 259.4 | 300.0 | 300.0 |
| Disintegrant | 12.0 | 20.0 | 25.0 |
| Total | 500.0 | 1077.25 | 1132.25 |

EXAMPLE 2

The following procedure indicates the cytoprotective action of various metal bases or basic salts when administered concomitantly with the non-steroidal anti-inflammatory agent.

To each of a group of 10 Wistar rats was administered the indicated dosage of the specified metal base or basic salt together with indomethacin at a dose of 20 mg/kg in 0.25% of methylcellulose. Water was withheld for six hours and the animals then were sacrificed with carbon dioxide inhalation and the gastric mucosa of the stomachs examined.

A group of ten animals served as the placebo control and received indomethacin in 0.25% of methylcellulose at a dose of 20 mg/kg but with no cytoprotective agent.

| Test Compound | Dose (mg/kg) | No. of Ulcers | % Change |
|---|---|---|---|
| Placebo Control | 0 | 91 | -- |
| Magnesium Silicate | 10 | 34 | -63 |
| Aluminum Hydroxide | 10 | 49 | -46 |
| Aluminum Hydroxide | 5 | 49 | -46 |
| Aluminum Hydroxide | 2.5 | 51 | -44 |
| Bismuth Subcarbonate | 10 | 66 | -27 |
| Pepto Bismol[R] | 0.5 ml/kg | 59 | -35 |
| Sodium Bicarbonate | 10 | 25 | -73 |

As can be seen from the above, all of the metal bases or basic salts produced a significant reduction in ulcer formulations.

EXAMPLE 3

The following procedure indicates the desirability of administering the cytoprotective metal base or basic salt in temporal proximity with the nonsteroidal anti-inflammatory agent. To each of a group of 10 Wistar rats was administered the indicated dose of the metal base or basic salt at 8 a.m., 12 noon, and 4 p.m. for two days. The test animals were fasted on the second day. On the third day (following 24 hours of fasting) the animals were challenged with indomethacin at a dose of 20 mg/kg without administration of a cytoprotective metal base or basic salt. Water was withheld for six hours and the animals then were sacrificed and the gastric mucosa of the stomachs examined.

A second group of ten animals received indomethacin at a dose of 20 mg/kg on day 3 but received no pretreatment. Third and fourth groups, each of 10 animals, received vehicle controls of 0.25% methylcellulose or 0.5 ml/kg of Pepto-Bismol[R] and 0.25 methylcellulose, t.i.d. on days 1 and 2.

| Test Compound | Indomethacin | No. of Ulcers | % Change |
|---|---|---|---|
| 0.25% methylcellulose | 0 | 0 | -- |
| Pepto Bismol[R] | 0 | 0 | -- |
| --- | 20 | 57 | -- |
| Magnesium Hydroxide | 20 | 24 | -58 |
| Aluminum Hydroxide | 20 | 66 | +16 |
| Aluminum Hydroxide[1] | 20 | 71 | +25 |
| Aluminum Hydroxide[2] | 20 | 40 | -30 |
| Bismuth Subcarbonate | 20 | 55 | -4 |
| Pepto Bismol[R] | 20 | 78 | +37 |
| Sodium Bicarbonate | 20 | 73 | +28 |

1. $Al(OH)_3$ at 5 mg/kg.
2. $Al(OH)_3$ at 2.5 mg/kg

As can be seen from the above, magnesium hydroxide effected a 58% reduction in ulcer formation caused

4

by the non-steroidal anti-inflammatory agent. At the same dose, aluminum hydroxide, sodium bicarbonate and Pepto Bismol[R] all increased ulcer formation of the non-steroidal antiinflammatory agent. Aluminum hydroxide at a dose of 2.5 mg/kg caused a reduction in ulcer formation; bismuth subcarbonate also caused a reduction which was not statistically significant.

The present invention, having thus been described, is summarised by the following clauses, numbered 1-9, and is defined by the claims appended thereafter.

1.In the method of treating inflammation by administration in a multiple dose regimen of a non-steroidal systemic anti-inflammatory agent which inhibits biosynthesis of prostaglandins and selected from the group consisting of fenamic acid derivatives, indene derivatives, and ibufenac derivatives, the improvement which comprises administering said anti-inflammatory agent in close temporal proximity to administration of a nontoxic prostaglandin-stimulating metal base or basic salt.

2. The method of 1, wherein the metal base or basic salt is a hydroxide, sulfate, carbonate, bicarbonate, subcarbonate, or trisilicate.

3. The method of 1, wherein the metal base or basic salt is an aluminum, sodium, magnesium, potassium, or bismuth base or basic salt.

4. The method of 1, wherein the metal base or basic salt is aluminum hydroxide.

5. The method of 1, wherein the metal base or basic salt is magnesium silicate.

6. The method of 1, wherein the non-steroidal systemic anti-inflammatory agent is selected from the group consisting of flufenemic acid, mefenamic acid, meclofenamic acid, clonixeril, clonixin, flunixin, diclofenac, and the pharmaceutically acceptable salts thereof.

7. The method of 6, wherein the non-steroidal systemic anti-inflammatory agent is meclofenamic acid or a pharmaceutically acceptable salt thereof.

8. The method of 1, wherein the non-steroidal systemic anti-inflammatory agent is selected from the group consisting of indomethacin,carprofen, etodolac, fendosal, indoprofen, prodolic acid, sermetacin, zidometacin, zomeprirac, tolmetin, sulinac, cicloprofen, cinchophen, and the pharmaceutically acceptable salts thereof.

9. The method of 1, wherein the non-steroidal systemic anti-inflammatory agent is selected from the group consisting of diflunisal, fenoprofen, ibuprofen, naproxen, alclofenac, amfenac, cliprofen, fenclofenac, fenclorac, fluprofen, ketoprofen, naproxol, fenbufen, ibufenac, and the pharmaceutically acceptable salts thereof.

## Claims

1. The use of a non-toxic prostaglandin-stimulating metal base or basic salt in the manufacture of a medicament for the treatment of inflammation.

2. The use according to claim 1, in which the metal base or basic salt is a hydroxide, sulfate, carbonate, bicarbonate, subcarbonate, or trisilicate.

3.. The use according to any preceding claim, wherein the metal base or basic salt is an aluminum, sodium, magnesium, potassium, or bismuth base or basic salt.

4. The use according to any preceding claim, wherein the metal base or basic salt is aluminum hydroxide.

5. The use according to any one of claims 1-3, wherein the metal base or basic salt is magnesium silicate.

6. A medicament, for the treatment of inflammation, as obtained from the use according to any one of the preceding claims.

7. The use according to any one of claims 1 to 5, wherein the medicament is administered in close temporal proximity to a multiple dose regimen of a non-steroidal systemic anti-inflammatory agent which inhibits biosynthesis of prostaglandins and which is selected from the group consisting of fenamic acid derivatives, indene derivatives, and ibufenac derivatives.

8. The use according to claim 7, wherein the medicament is administered in close temporal proximity to a multiple dose regimen of a non-steroidal systemic anti-inflammatory agent selected from the group consisting of flufenemic acid, mefenamic acid, meclofenamic acid, clonixeril, clonixin, flunixin, diclofenac, and the pharmaceutically acceptable salts thereof.

9. The use according to claim 7, wherein the medicament is administered in close temporal proximity to a multiple dose regimen of meclofenamic acid or a pharmaceutically acceptable salt thereof.

10. The use according to claim 7, wherein the medicament is administered in close temporal proximity to a multiple dose regimen of a non-steroidal systemic anti-inflammatory agent selected from the group consisting of indomethacin, carprofen, etodolac, fendosal, indoprofen, prodolic acid, sermetacin, zidometacin, zomeprirac, tolmetin, sulinac, cicloprofen, cinchophen, and the pharmaceutically acceptable salts thereof.

**11.** The use according to claim 7, wherein the medicament is administered in close temporal proximity to a multiple dose regimen of a non-steroidal systemic anti-inflammatory agent selected from the group consisting of diflunisal, fenoprofen, ibuprofen, naproxen, alclofenac, amfenac, cliprofen, fenclofenac, fenclorac, fluprofen, ketoprofen, naproxol, fenbufen, ibufenac, and the pharmaceutically acceptable salts thereof.

**12.** A kit of parts useful in the treatment of inflammation, comprising:

a) the medicament according to claim 6;

b) a multiple dose regimen of a non-steroidal systemic anti-inflammatory agent selected from the group consisting of fenamic acid derivatives, indene derivatives, and ibufenac derivatives; and

c) instructions for the close temporal administration of (a) and (b).